# EUROPEAN PATENT APPLICATION

(11) **EP 4 799 632 A1**
(43) Date of publication of application: **02.09.2026**
(21) Application number: 25160769.3
(22) Date of filing: 27.02.2025
(51) Int. Cl.: A61K 33/00, A61K 45/06, A61M 1/00, A61P 29/00, A61P 43/00

(54) **GAS COMPOSITION COMPRISING OXYGEN AND/OR OZONE FOR USE IN REDUCING INFLAMMATION**

(71) Applicant: Sangair AB, 171 65 Solna (SE)
(72) Inventor: Rundgren, Henrik, Solna (SE); Sjöholm, Johan, 224 80 Lund (SE)
(74) Representative: KIPA AB

(57) **Abstract**

A gas composition comprising oxygen and/or ozone is provided for use in treatment to reduce inflammation in conditions characterized by dysregulated immune response, to preserve organ function in conditions characterized by dysregulated immune response, and to protect the endothelium or restore endothelial function in conditions characterized by dysregulated immune response. Said use of the gas composition comprising oxygen and/or ozone reduces one or more pro-inflammatory cytokines, thereby reducing inflammation.

## Description

### Field of the Invention

The present invention relates to a gas composition comprising oxygen and/or ozone for use in reducing inflammation in conditions characterized by dysregulated inflammation.

### Background of the Invention

Bacterial infections may progress to sepsis and septic shock, conditions associated with substantial mortality rates. Antibiotic treatment is crucial for severe infections, but global antibiotic resistance is increasing and is now classified as one of the top ten global public health risks facing humanity by the World Health Organization.

Sepsis is associated with substantial mortality rates. Traditional treatment strategies often fail to address the underlying dysregulation in immune response, necessitating novel therapeutic approaches.

Sepsis is a life-threatening condition in which the body's own immune system overreacts in response to a bacterial, viral or fungal infection infection. According to recent studies, sepsis is the third leading cause of death, accounting for 20% of all global deaths. In Sweden, the mortality rate for sepsis patients in intensive care units is approximately 30%.

Sepsis is difficult to treat, partly because of the broad individual presentation of sepsis, and partly because of the different types of bacteria and viruses that cause the immune response and hence sepsis.

Sepsis is characterized by a dysregulated immune response which leads to organ dysfunction. Traditional treatment strategies often fail to address this underlying dysregulation, necessitating novel therapeutic approaches. Immunomodulatory therapy holds promise by restoring immune balance and mitigating excessive inflammation.

According to sepsis-3, the latest and updated version, sepsis is defined as severe multiple organ failure due to an infection. The old definition, which included fever and a positive infection test, is no longer used. Today, it is sufficient that the patient requires organ support for at least two organ systems in the event of a suspected infection. This should allow sepsis to be diagnosed at an earlier stage.

Despite an earlier diagnosis, the treatment of sepsis is still very complicated and must be individually adapted. Patients need organ support according to which organs are failing as well as antibiotics/antivirals to treat the infection itself. The body's immune system is extremely fast and effective at eliminating bacteria from the blood system, which is why the majority of patients have negative blood cultures. Sepsis does not mean that bacteria are present in the blood, but sepsis is the body's own immune system that attacks the organs through an overreaction induced by bacteria or viruses. It is therefore the body's own immune system that causes the actual organ damage.

With today's standard treatment for sepsis, a patient is treated with antibiotics/antivirals for the infection and organ support for the failing organs - i.e. not the overreactive immune system. For example, mechanical ventilation for lung failure, blood pressure medications for low blood pressure, dialysis for kidney failure, etc. Therefore, there is a gap-of-knowledge in research on how to modulate the harmful and overreactive immune system.

The majority of the new methods being researched to modulate the immune system in sepsis have an on/off effect. The pills or liquid medications have the effect of either shutting down the immune system during a dysregulated immune response or not affecting the dysregulated immune response. Thus, these pills or liquid medications have failed to show significant efficacy in sepsis.

The body's immune system must always remain functional; however, the immune system needs to function within balanced parameters, neither overreacting nor underreacting. for the body to be able to eliminate the infection in sepsis, without, or at least minimizing, the negative repercussions on the body's own organs.

Therefore, there is a need for developing a personalized, controllable brake on the immune system.

Ozone (O₃) is an inorganic molecule with no evident function in the body. It is a strong oxidizing agent that has been used as a disinfectant. Despite the corrosive and highly reactive nature of ozone, the use of ozone to kill or inactivate certain infectious agents in blood has been explored.

For example, US 2005/0189302 A1 discloses a method of inactivating viruses in blood by exposing the blood to ozone in a gas-fluid contacting device that maximizes gas-fluid mass transfer. The contacting device contains spheres or rods to form a thin film of blood and treatment is preferably performed at ambient temperature. WO 2011/162805 A2 discloses methods for inactivating infectious prion proteins in blood by subjecting blood to an ozone/oxygen admixture using a gas-fluid contacting device similar to that disclosed in US 2005/0189302 A1.

WO 93/15779 A1 discloses a method of increasing the nitric oxide concentration in the blood by contacting a sample of blood with ozone gas and ultraviolet radiation. The treated sample of blood is administered to a patient to treat a variety of conditions including bacterial, fungal, viral, and protozoal infection. The blood sample is most preferably a volume of 1 to 50 mL and is most preferably treated at a temperature of 42.5°C for approximately 3 minutes.

US 6,027,688 discloses a method for inactivating viruses, bacteria, fungi and protozoa in blood by contacting a flow of blood with counter-flow of an ozone-oxygen mixture for about 16 seconds using a gas-liquid contact apparatus.

WO2004/041314 A1 relates to using ultrasound for cancer therapy and discloses that emission of microbubbles combined with ultrasound and optionally light radiation is effective at inactivating and removing hyperproliferative cells in, for example a physiological fluid.

WO2016043649A1 (SangAir's application) relates to methods and apparatus for treating biological fluids with ozone, more particularly, to methods and apparatus for treating blood with ozone.

There is a need for uses and an apparatus to treat and prevent immune dysregulation.

### Summary of the Invention

Embodiments of the present invention preferably seek to mitigate, alleviate or eliminate one or more deficiencies, disadvantages or issues in the art, such as the above-identified, singly or in any combination by providing uses and an apparatus to treat and prevent immune dysregulation.

The invention is disclosed in the appended independent patent claims. Some particular embodiments of the invention are defined in the appended dependent claims.

According to one aspect of the disclosure a gas composition comprising oxygen and/or ozone is provided for use in treatment to reduce inflammation in conditions characterized by dysregulated immune response.

According to a second aspect of the disclosure, a gas composition comprising oxygen and/or ozone is provided for use in treatment to preserve organ function in conditions characterized by dysregulated inflammation.

According to a third aspect of the disclosure, A gas composition comprising oxygen and/or ozone is provided for use in treatment to protect the endothelium or restore endothelial function in conditions characterized by dysregulated inflammation.

According to a fourth aspect of the disclosure, a gas composition for use comprising oxygen and/or ozone is provided for use in treatment to reduce inflammation in conditions characterized by dysregulated inflammation, wherein said gas comprises 0-10% ozone, and said one or more pro-inflammatory cytokines selected from the group consisting of IL-1β, IL-6, IL-8, TNF-α and IFN-γ, by at least 1% optionally comprising or more further agents selected from the group consisting of one or more antimicrobial agents and/or immunomodulators. In the gas composition said gas is administered/contacted with blood by means of an apparatus, an ozonation chamber (2), a blood flow inlet (2a), and a blood flow outlet (2b) wherein, said ozonation chamber (2) comprises an ozone injector (3) , said ozone injector (3) configured for connection to a source of ozone containing gas and to inject bubbles of ozone containing gas into a flow of blood through the ozonation chamber (2), wherein the flow of blood is vertically upwards through the blood ozonation chamber (2) during injection. Said ozonation chamber (2) and said ozone injector (3) are configured such that the vertically upwards flow of blood through the blood ozonation chamber (2) entrains said bubbles of ozone containing gas into said flow of blood, wherein, a blood processing unit (13) comprising means for removing foam and bubbles from the flow of blood downstream the ozone injector (3). Said ozone injector (3) is a porous ozone injector, and said ozone injector is configured to inject microbubbles having a mean diameter of less than 5 µm, thereby reducing inflammation.

Unless otherwise defined, all terms used herein, including technical and scientific terms, have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The term "microorganism" as used herein is synonymous with "microbe" and means any microorganism including bacteria, virus, fungi or yeast, and including spores or dormant forms of such a microorganisms.

The term "dysregulated immune response" as used herein means an immune response that is directed against one of the body's own tissues, cells or molecules (eg. autoimmunity), and may comprise dysregulated inflammation, such as exaggerated inflammation response.

The term "antimicrobial agents" as used herein means any agent that destroys microorganisms or suppresses their multiplication or growth

The term "immunomodulators" as used herein means drug treatments that change a body's immune response.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a schematic of a first embodiment of an ozonation system comprising an apparatus for contacting blood with ozone.
Fig. 2 is a schematic of a second embodiment of an ozonation system comprising an apparatus for contacting blood with ozone.
Fig. 3 shows the experimental setup.

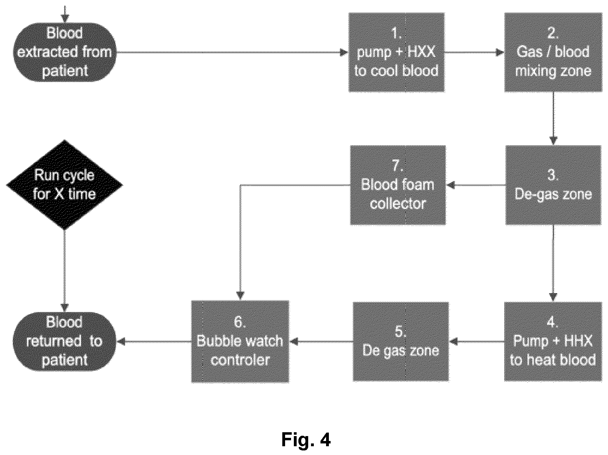

Fig. 4 shows the steps involved in the ozonation of blood with a blood ozonation apparatus.
Fig. 5A shows the effect of a single pass treatment decrease in viable P. *aeruginosa.*
Fig. 5B shows bacterial concentration between groups detected in peripheral venous blood.
Fig. 5C shows TNFa levels; Fig. 5D, IL-1β levels; Fig. 5E, IL-2 levels; Fig. 5F, IL-4 levels; Fig. 5G, IL-6 levels; Fig. 5H, IL-8levels; Fig. 5I, IL-10 levels; and Fig. 5J, IFN-γ levels.
Figure 5K-L shows complement function, classic pathway and alternative pathway, compared to baseline.
Fig. 6A-6H6I shows comparisons of circulatory effects from the sepsis induction and the ozone treatment.
Fig. 7A-7I show comparisons of respiratory effects from the sepsis induction and the ozone treatment.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

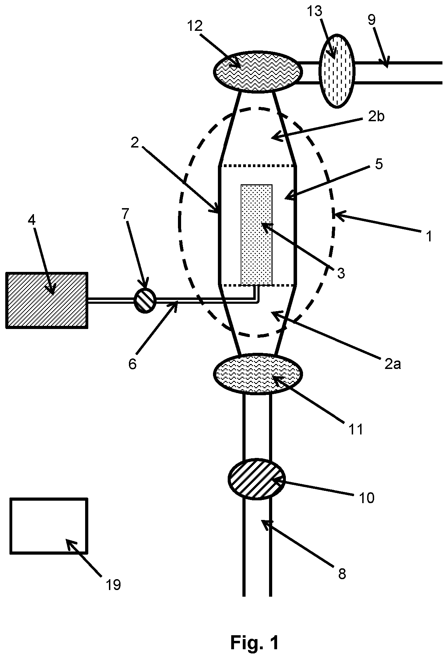

An example of an ozonation system is shown in **Fig. 1**. The apparatus comprises an ozonation apparatus 1 comprising an ozonation chamber 2 comprising an inlet 2a and an outlet 2b, and an ozone injector 3. The ozone injector 3 is configured to be connected to a source of ozone gas 4.

The source of ozone gas may be an ozone generator that produces ozone from oxygen that may produce a gas mixture that contains ozone and oxygen at a certain ratio. Alternative gaseous ozone sources may be provided as known to the skilled person.

The ratio of ozone to oxygen in the gas mixture may for example range from 5% to 20% ozone and from 95% to 80% oxygen. Preferable values of an ozone to oxygen ratios are 5/95, 10/90, 15/85 and 20/80.

The ozone injector may be made from porous material, for example, from sintered stainless steel. Alternatively or in addition, it may be made from sintered ceramic. Alternatively, or in addition other gas injectors may be provided to provide ozone gas to enrich a flow of blood to be ozonized. Other gas injectors may be nozzle based. The porous material has in examples a uniform mean pore size of less than 5um in diameter, such as 4um, 3um, 2um, 1um, 0.5um, or 0.2um. The pore size is preferably between about 0.2um and about 2.0 um.

The ozone gas injector 3 may further comprises a flow meter, pressure valve, pressure sensor, and/or controlling element to facilitate monitoring and/or control the pressure and/or flow rate of gas being injected.

The example shown in Fig. 1 shows an ozonation apparatus 1 comprising a porous ozone injector 3 inside an ozonation chamber 2. The ozone injector is cylindrical in the example. The ozonation chamber is configured to guide a flow of blood around the porous microbubble releasing surface of the ozone injector 3. In the example, the ozone injector is cylindrical. Preferably, the cylindrical ozone injector 3 is arranged co-axially in the cylindrical ozonation chamber for advantageous ozonation efficiency. A gap exists between the inner wall of the ozonation chamber 2 and the porous bubble releasing surface of the ozone injector 3. The bubble as preferably microbubbles as elucidated herein.

The flow of blood over the microbubble releasing surface is preferably a laminar flow to minimize turbulence in the blood flow.

The blood flow is preferably directed vertically upwards so that gas bubbles entrain in the flow of blood. By directing the flow of gas in a vertical direction, gas bubbles are entrained upwardly. The gas bubbles are also provided to the entire flow of blood, independent of gravity. The gas bubbles are for instance evenly provided around a cylindrical ozone injector. In this manner, portions of blood that are not enriched with gas comprising ozone are avoided. The vertical direction provides thus advantageous (short) time of gas comprising ozone with the flow of blood until the gas is dissolved in the blood. This is important as for instance remaining gas bubbles returned to a patient have to avoided. In addition, foaming of the blood flow is advantageously reduced or avoided by the vertical arrangement of the blood flow.

Compared to e.g. horizontal arrangements as described in WO2008/066470, where portions liquid flow below an injector are not well or not at all ozonized as the bubbles raise upwardly along the injector, i.e. the flow portion above the injector is better ozonized than that below.

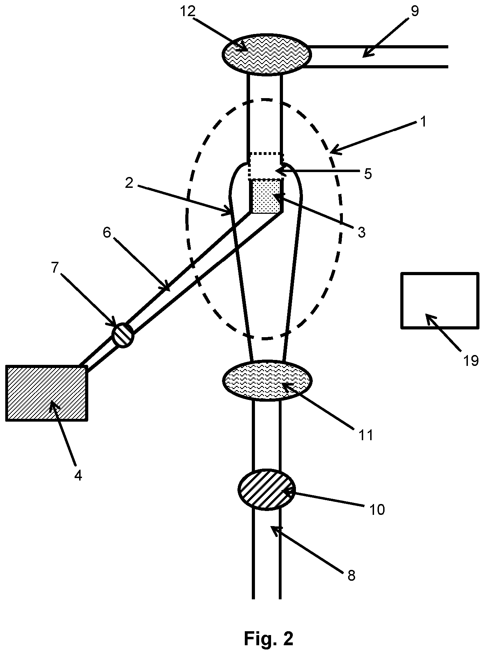

The shape of the ozone injector 3 need not be cylindrical and not all surfaces of the ozone injector need to be in contact with blood. One alternative configuration is shown in **Fig. 2****.**

The ozone injector 3 is arranged in the ozonation chamber 2 such that blood flow is restricted to pass through ozonation chamber 2 within a maximum distance (d) of a porous surface of the ozone injector 3 from which microbubbles are released. The maximum distance (d) is preferably less than about 2mm, such as 1.5mm, 1.0mm, 0.75mm, 0.5mm, 0.25mm, or 0.1mm or any distance within this range.

The ozonation chamber 2 and ozone injector 3 are designed to provide an ozonation zone 5 in which micrometer sized bubbles of ozone containing gas mix with and dissolve in a flow of blood.

When blood and ozone containing gas are flowing through the ozonation chamber 1, an ozonation region 5, indicated as the region between the horizontal dashed lines in **Fig. 1****,** exists in which microbubbles are entrained in the blood flow. The bubbles are dissolved in the blood along the entrainment path in the blood flow.

The duration of ozone contacting time, or dwell time, is determined by a number of factors. These factors include the concentration of ozone in the gas, and the flow rate of the gas. Another factor is the temperature of the blood. The temperature at ozonation is preferably controlled in examples of the disclosure. Moreover, the flow rate of the blood is such a factor. Any or all of these factors may be measured, supervised, or controlled to optimize ozonation. The factors maybe computer controlled by controlling gas pressure and flow from the source of ozone 4. Alternatively, or in addition, the operation of blood pumping means 10 may be controlled. Optionally blood cooling means 11 are controlled for advantageous blood ozonation.

A system according to an example of the present disclosure may comprise more than one ozonation apparatus. For example, a plurality of ozone apparatus may be arranged in parallel with respect to the flow of blood.

Alternatively, or in addition, a single ozone apparatus can comprise a plurality of ozone injectors. Each or some of the plurality of ozone injectors may be arranged such that all blood moving through the ozonation chamber passes within the maximum distance (d) of at least one of the ozone injectors.

The ozonation system shown in Fig. 1 additionally may, comprise, as illustrated, a blood pumping means 10. The blood pumping means 10 is arranged for pumping blood through the apparatus or system from a blood inlet portion 8 to a blood outlet portion 9. Suitable blood pumping means 10 for pumping blood are known in the art and may comprise, for example, a peristaltic pump, a roller pump, or a centrifugal pump. Pumping means 10 may be capable of pumping blood through the system at a suitable flow rate. Suitable flow rates range for example from about 0.1 Liters/hour to about 12 Liters/hour.

The system may also comprise an ozone source 4, such as a medical grade ozone generator. The ozone generator is fluidly connected to the ozone injector 3 by a gas conduit 6. The gas conduit 6 may comprise a one-way valve 7 to prevent backflow of blood into the ozone generator 6. Such backflow may occur in case of, for example, a sudden pressure drop in the gas conduit 6.

The ozone source 4 is preferably capable of delivering ozone at a constant pressure. The ozone source 4 is preferably capable of delivering at least 10% ozone at such a constant pressure. The constant pressure of gas is at least 0.1 bar and preferably up to 0.5 bar, 0.75 bar or 1.0 bar at the ozone injector 3.

The system may additionally comprise a source of one or more inert gasses that may be mixed with the ozone containing gas before reaching the ozone injector. Examples of inert gasses include nitrogen and helium.

The blood contacting portions of the system may preferably be made of any suitable biocompatible materials. Such materials include materials typically used to transfer or store blood, such as those used in tubing for transfusions or blood conducting portions of heart and lung machine or apheresis machines.

The blood entry portion 2 may comprise or be connectable to means for receiving blood from a patient such as a hollow needle for insertion into a vein.

Alternatively or additionally, the blood entry portion 2 may comprise means for receiving blood from a container of blood. Such container may include a bag or bottle or an extracorporeal blood circulation device such as an apheresis machine, dialysis machine, or heart and lunch machine.

The ozonation system may comprise blood cooling means 11 positioned upstream of ozonation apparatus inlet 2a and configured to cool the blood to a chilled temperature of less than 12°C before the blood enters the ozonation apparatus 1.

The blood cooling means 11 may comprise any suitable blood heat exchanger used in the medical arts to cool blood and may be configured for computer control of the temperature of the blood entering the ozonation chamber 2.

The blood cooling means 11 is preferably controllable to cool said blood to a temperature of between about 4°C and about 12°C, to produce chilled blood that is contacted with ozone containing gas in the ozonation chamber 2.

It is understood that cooling is a relative term. If for instance blood entering into the ozonation system has a temperature lower than desired, e.g. lower than between about 4°C and about 12°C, it may be provided to actually heat the blood to this preferred temperature range. This may be the case for frozen blood preservatives, e.g. frozen blood plasma. Cooling can be understood a term relative to ambient room temperature. Cooling is also relative physiological body temperature if blood is input to the ozonation system at body temperature from a patient.

At ozonation in the ozonation chamber, the blood thus has preferably a temperature of less than 12°C. It preferably has a temperature between about 4°C and about 12°C. The chilled blood is then contacted at this temperature with ozone containing gas in the ozonation chamber 2.

Additionally or alternatively, the ozonation system may comprise blood warming means 12. The blood warming means is preferably positioned downstream the ozonation chamber. The blood warming means is for example positioned downstream of the outlet 2b from the ozonation apparatus 1. The blood warming means is configured to warm ozonated blood to a temperature above the blood temperature in the ozonation chamber 2.

The blood warming means 12 may be configured to warm blood to a body temperature of a human or nonhuman animal, e.g. between about 24°C to about 39°C.

Blood warming means 12 may additionally or alternatively be configured to adjust the temperature of the blood to a storage temperature. It is understood that warming is a relative term. If for instance blood leaving the ozonation system has a temperature higher than desired to exit the apparatus or system, it may be provided to actually, potentially further, cool the blood to this desired temperature. This may be the case when it is desired to provide ozonated frozen blood preservatives, e.g. frozen blood plasma, with advantageous storage properties compared to not ozonated blood products.

Blood warming will in most examples include feeding heat energy to the ozonated blood to increase the temperature thereof.

Blood warming means 12 may be computer controlled similarly to blood cooling means 11.

The system may comprise a blood processing unit 13 comprising means for removing bubbles, preferably downstream the ozone injector. Alternatively, or in addition, the system may comprise a blood processing unit 13 comprising means for removing foam, preferably downstream the ozone injector. Alternatively, or in addition, the system may comprise a blood processing unit 13 comprising means for removing thrombi, preferably downstream the ozone injector. In this manner, undesired bubbles, foam, thrombi, and/or other material may be suitably removed. The treatment may be at least partially be done upstream the ozonation chamber. The treatment is preferably done downstream (relative blood flow) the ozonation chamber, for instance before the blood can be delivered to a patient or to a blood storage container.

Blood processing unit 13 may comprise, for example, a filter for removing particles. Alternatively, or in addition, the blood processing unit 13 may comprise a blood reservoir comprising an inlet region separated from an outlet region by a membrane that is permeable for blood, but impermeable for air bubbles.

A blood processing unit 13 downstream the ozonation chamber may be particularly useful in embodiments of the invention used to ozonate blood that is to be delivered to a patient.

All or parts of the apparatus and/or system may be combined with and/or controlled by a computerized control system 19. The computerized control system 19 may be electrically coupled to sensors, actuators, valves, and/or switches in the ozonation system.

The control system 19 may comprise software having code segments configured to control operational parameters of the apparatus. Operational parameters include parameters such as blood and gas flow rates and pressures, the concentration of ozone and other gasses in the ozone containing gas, blood temperature in the different portions of the apparatus, and blood flow rates in various portions of the apparatus and/or system conduit based upon sensor data received from pressure, flow, temperature, and/or chemical sensors provided in various portions of the apparatus.

Fig. 2 shows an example of an ozonation system comprising an alternatively configured ozonation apparatus 1. Only the top surface of the ozone injector 3 is in contact with blood and injects microbubbles of ozone containing gas into a flow of blood that entrains the injected microbubbles. An ozonation region 5 is located directly above the ozone injector 3 in which the microbubbles completely dissolve in the blood.

Without intending to be bound by theory, it is believed that the ozone is also entirely consumed by chemical reactions with the blood within the ozonation region 5.

The ozonation chamber 2 is configured such that all of the blood flow passes within a maximum distance (d) from the upper surface of the ozone injector 3. The maximum distance (d) is preferably less than about 2mm, such as 1.5mm, 1.0mm, 0.75mm, 0.5mm, 0.25mm, or 0.1mm or any distance within this range.

Alternatively, the diameter of blood flow can be narrowed above the ozone injector 3 such that a flow of blood entrains a flow of microbubbles in an ozonation zone 5 above the injector.

With such an embodiment, the maximum distance from the surface of the ozone injector 3 may be greater than in other embodiments. The diameter of the flow in the ozonation zone 5 is preferably less than about 2mm, such as 1.5mm, 1.0mm, 0.75mm, 0.5mm, 0.25mm, or 0.1mm or any distance within this range.

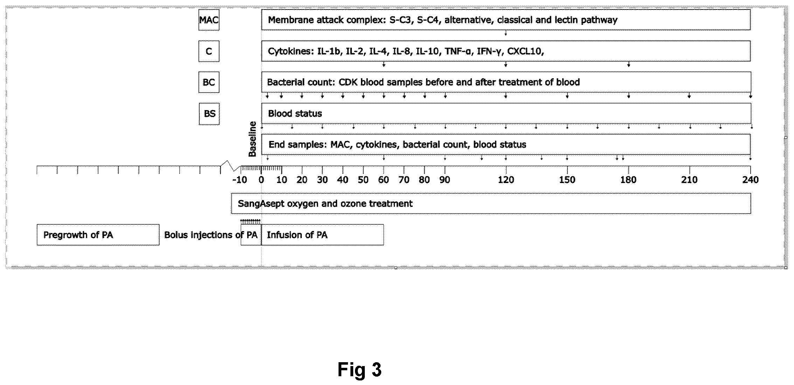

A method for contacting blood with ozone (Fig. 3) comprises receiving a flow of blood 301 into an ozonation system. The method may include injecting microbubbles of ozone containing gas 303 into the flow of blood. Further, the method may include delivering the ozonated blood from the system 305. Preferably, the blood is at a temperature of less than 12°C during the injecting of ozone. Ozone containing gas is preferably provided as microbubbles that have a mean diameter of less than 5 um. The temperature of the blood during ozone injection is preferably between about 4°C and about 10°C, such as 5°C, 6°C, 7°C, 8°C, or 9°C.

The ozone containing gas is normally produced by an ozone generator that produces a mixture of ozone and oxygen comprising between 5% and 20% ozone, a range and ratio as described above.

The concentration of ozone in the ozone containing gas injected into the flow of blood is preferably less than 10 grams/m³ and more preferably less than 5 grams/m³. Preferably, the concentration of ozone in the ozone containing gas is at least 1 gram/m³.

The pressure of gas being injected may be for example, at least 0.1 bar and preferably up to 0.5 bar, 0.75 bar or 1.0.

The ratio of the volumetric flow rate of blood and the volumetric flow rate of ozone containing gas may be adjusted to be in a range of from about 10:1 to about 50:1.

The volumetric flow rate of blood may be in the range of from 0.1 Liters/hour to 12 Liters/hour, for example 3 Liters/hour to 6 Liters/hour.

The delivered dose of ozone in the blood flow is less than 10 ppm, and may be for example, 1 ppm, 2 ppm, 3 ppm, 4 ppm, 5 ppm, 6 ppm, 7 ppm, 8 ppm, or 9 ppm.

The flow of blood is preferably a laminar flow to minimize turbulence.

The flow rates and pressures are controlled or set such that the microbubbles have a substantially uniform mean diameter of less than 5um in diameter.

The flow rates and pressures are controlled or set such that the microbubbles completely dissolve in the flow of blood in less than 10 seconds, preferably less than 5 seconds, and more preferably less than 2 seconds.

Controlling the flow and pressure of the ozone containing gas and blood flow rate in this way can prevent the microbubbles from coalescing into larger bubbles so that essentially all of the microbubbles entrained in the blood flow have the desired diameter.

If the method is to be performed on blood from a patient or other source of blood that has a temperature higher than the ozonation temperature, the method may further comprise cooling the blood 302 before ozone is injected into the flow of blood 303.

If the blood is to be returned to a patient or stored at a temperature above the ozonation temperature, the blood may be warmed 304 before being delivered from the system 305.

If the ozonated blood is to be stored, warming the blood may not be necessary, or alternatively cooling is provided as describe above.

The flow of blood received into the ozonation system may optionally be pre-treated 301a, for instance, by a blood processing unit upstream the ozonation location. Alternatively or in addition, for example, an anticoagulant or other drug may be added to the blood flow. Alternatively, or in addition, addition of such substance may be provided to ozonated blood, i.e. downstream the ozonation location.

Extracorporeal treatment of blood introduces a possible undesired blood tampering, such as bubble formation, blood foaming, and the formation of clots or precipitates. The method may therefore include one or more post treatment processing steps 304a to remove foam, bubbles, clots, and/or precipitates, as described above.

Blood ozone contacting time and temperature may be optimized for killing particular microorganisms, or microbes.

The contacting may be comprise the administration of drugs that may have an additive or synergistic effect with the ozone to kill microbes and/or a protective effect to reduce unwanted or adverse effects associated with contacting blood with an ozone containing gas.

A gas composition comprising oxygen and/or ozone is provided for use in treatment to reduce inflammation in conditions characterized by dysregulated immune response. The gas composition may comprise 0-20% ozone, preferably 0 - 10% ozone, and from 100% to 80% oxygen. Preferably, the gas composition may consist of oxygen and/or ozone.

The use of the gas composition may reduce one or more pro-inflammatory cytokines, thereby reducing inflammation. The cytokines may be selected from the group consisting of IL-1β, IL-6, IL-8, TNF-α and IFN-γ. The one or more cytokines may be reduced by at least 1%, such as at least 5%, such as at least 8%, at least 10%, at least 12%, or for example in the range from 1 to 80%, such as 5 to 80%.

In the gas composition the conditions characterized by dysregulated immune response may be either secondary to an infectious trigger, or secondary to a non-infectious trigger. Preferably, the non-infectious trigger may be selected from the group consisting of trauma, tissue damage, multiple sclerosis, autoimmune and low-grade inflammatory conditions.

A gas composition comprising oxygen and/or ozone is provided for use in treatment to preserve organ function in conditions characterized by dysregulated immune response.

A gas composition comprising oxygen and/or ozone is provided for use in treatment to protect the endothelium or restore endothelial function in conditions characterized by dysregulated immune response.

The use of the gas composition may comprise at least one additional agent such as one or more selected from the group consisting of one or more antimicrobial agents and/or immunomodulators.

The gas composition may be administered/contacted with blood by means of an apparatus comprising an ozonation chamber (2), a blood flow inlet (2a), and a blood flow outlet (2b).

Preferably, the apparatus may comprise an ozonation chamber (2), a blood flow inlet (2a), and a blood flow outlet (2b), wherein, said ozonation chamber (2) comprises an ozone injector (3) , said ozone injector (3) configured for connection to a source of ozone containing gas composition and to inject bubbles of ozone containing gas into a flow of blood through the ozonation chamber (2), wherein the flow of blood is vertically upwards through the blood ozonation chamber (2) during injection. Said ozonation chamber (2) and said ozone injector (3) may be configured such that the vertically upwards flow of blood through the blood ozonation chamber (2) entrains said bubbles of ozone containing gas into said flow of blood, wherein, a blood processing unit (13) comprising means for removing foam and bubbles from the flow of blood downstream the ozone injector (3); said ozone injector (3) may be a porous ozone injector, and said ozone injector may be configured to inject microbubbles having a mean diameter of less than 5 µm.

Said apparatus may be configured for extra-corporeal treatment of blood.

The extracorporeal treatment of blood may be performed continuously, or in intermittent periods.

Said treatment of said blood may be made prior to storage of said blood and provides enhanced storage time of said ozonated blood compared to said blood when not treated.

Said in vitro treatment of said blood may be made after storage of said blood and/or before administration of said blood to a patient.

A gas composition for use comprising oxygen and/or ozone is provided for use in treatment to reduce inflammation in conditions characterized by dysregulated immune response, wherein said gas comprises 0-10% ozone, wherein said one or more pro-inflammatory cytokines selected from the group consisting of IL-1β, IL-6, IL-8, TNF-α and IFN-γ, by at least 1%, optionally comprising one or more further agents selected from the group consisting of one or more antimicrobial agents and/or immunomodulators wherein said gas is administered/contacted with blood by means of an apparatus, an ozonation chamber (2), a blood flow inlet (2a), and a blood flow outlet (2b) wherein, said ozonation chamber (2) may comprise an ozone injector (3), said ozone injector (3) configured for connection to a source of ozone containing gas and to inject bubbles of ozone containing gas into a flow of blood through the ozonation chamber (2), wherein the flow of blood is vertically upwards through the blood ozonation chamber (2) during injection. Said ozonation chamber (2) and said ozone injector (3) may be configured such that the vertically upwards flow of blood through the blood ozonation chamber (2) entrains said bubbles of ozone containing gas into said flow of blood, wherein, a blood processing unit (13) comprising means for removing foam and bubbles from the flow of blood downstream the ozone injector (3); said ozone injector (3) may be a porous ozone injector, and said ozone injector may be configured to inject microbubbles having a mean diameter of less than 5 µm, thereby reducing inflammation.

The present invention has been described above with reference to specific embodiments. However, other embodiments than the above described are equally possible within the scope of the invention as defined in the appended patent claims.

### Experimental Methods and Data

### Methods

The animal experiments were approved by the Animal Ethics Committee in Linköping, Sweden (approval no: 3264, 12578-2020 /17635-2020) and performed in agreement with the ARRIVE guidelines. The swine were handled in accordance with the Guide for the Care and Use of Laboratory Animals. The surgery was performed under general anesthesia and efforts were made to minimize suffering. Animals always had water ad libitum, and food until 12 hours before the experiment. 13 specific-pathogen-free (SPF) Yorkshire/Swedish landrace crossbred swine (6 female, 7 male) with a mean weight of 42 (range 39-46) kg were provided from Company Johansson, Stockholm Region, Sweden. The experimental setup is described in Figure 3.

### In vivo experiments

The animals were pre-medicated with 150 mg tiletamine/zolazepam (Zoletil 100 Vet) and 6 mg medetomidine (Cepetor) after which anesthesia was induced with 0.2-0.3 mg alfentanil, 100 µg fentanyl (50 µg/mL) and 120-240 mg pentobarbital (60 mg/mL). Endotracheal intubation was performed with a Miller-type laryngoscope using a standard cuffed size 7 tube. Throughout the study, perioperative hypnosis was maintained with fentanyl 200-300 µg/hour and pentobarbital 800 mg/h. The animals were ventilated with a Dräger ventilator using pressure control with initial settings positive end-expiratory pressure (PEEP) 5, peak inspiratory pressure (PIP) 15 cm H20, respiratory rate 15/minute and FiO2 always remained at 21%. Settings were adjusted to maintain normoventilation (PaCO₂ 4.9-5.7 kPa). A 7.5 F, 110 cm pulmonary artery catheter (Edwards Lifescience) was inserted into the right internal jugular vein via cut-down for continuous monitoring of central venous pressure (CVP), cardiac output (CO), pulmonary artery pressure (PAP), mixed venous oxygen saturation (SvO₂), pulmonary arterial wedge pressure (PAWP), and core temperature (Vigilance CEDV monitor, Edwards Lifescience). Arterial blood gas parameters, including hemoglobin (Hb), methemoglobin (MetHb), pH, PaCO₂, PaO₂, Na⁺, K⁺, Ca²⁺, glucose, lactate, hematocrit (Hct), SaO₂, and base excess, were measured at baseline and at regular intervals throughout the experiment (ABL90 FLEX Plus). A 13.5 F, 15 cm double-lumen dialysis catheter (MedCOMP) was placed in the right femoral vein to facilitate ozone therapy. Continuous perioperative monitoring included electrocardiography and urine output. An initial fluid bolus of 500 mL Ringer's acetate was administered at anesthesia induction to correct preoperative fluid imbalances, followed by a maintenance infusion rate of 3 mL/kg/h. Additional boluses of 100 mL were administered if mean arterial pressure (MAP) dropped below 35 mmHg. To simulate an intensive care environment, animals were managed according to a protocol aimed at maintaining vital parameters within normal physiological limits, consistent with prior studies (13) and clinical best practices for sepsis in an intensive care unit. This included the use of noradrenaline and vasopressin for hemodynamic instability if required (1).

After preparation, animals were assigned to one of two groups: *P*. *aeruginosaP. aeruginosa* infusion with ozone treatment (n = 7) or *P*. *aeruginosaP. aeruginosa* infusion without ozone treatment (n = 6). Live *P*. *aeruginosaP. aeruginosa* was infused through a peripheral vein catheter for 1 hour starting at baseline. To prevent clotting in the extracorporeal ozone system, 4000 IU of heparin was administered intravenously 15 minutes before ozone therapy began, followed by a continuous infusion of 33 IU/kg/h throughout the treatment period. This was also given in the group not receiving ozone treatment. At the end of the experiment, or at the time of circulatory collapse, the animals were euthanized with 40 mL pentobarbital sodium (Alfatal Vet 100 mg/mL).

### Extracorporeal blood ozonation

The SangAsept blood ozonation prototype (patent no. WO2016/043649) was used to treat blood with ozone. In this system, blood was drawn from the venous circulation of the swine at an average flow rate of 50 mL/min. The blood was first cooled to a median temperature of 4.1 °C, then exposed to a gaseous mixture of oxygen containing 3.1-4.7% ozone, as ozone solubility increases at lower temperatures. The ozone concentration was maintained below 100 g/m³, with a gas flow rate of 15 mL/min. This cooling step optimized the ozonation process. After treatment, the blood was warmed to an average temperature of 29.6 °C before being returned to the swine. For in vitro studies, the treated blood was instead collected in a separate container **(****Fig. 3****).**

### Organism

The bacteria strain, *P*. *aeruginosa* (PA-103, ATCC 29260, CCUG31589) was obtained from Uppsala University. The day before the experiment, bacteria from the master plate was streaked out on four Cystine Lactose Electrolyte Deficient agar plates (CLED) to form a lawn and incubated overnight at 37 °C. Bacteria were collected from the plates and used to inoculate 200 mL of Luria-Bertani broth that was grown under agitation at 37 °C for at least 90 minutes. Bacteria were pelleted by spinning the culture in 4x50 mL tubes at 2000xg (3549 RPM) for 5 minutes. The bacteria pellet resuspended in 4x10 mL NaCl. The bacteria suspension was centrifuged again as previously described, the supernatant removed, the bacteria resuspended and diluted in NaCl to OD=1.0. Thereafter, the bacteria suspension was diluted 1:2 in NaCl to an approximate concentration of 5x108 colony forming units (CFU)/mL.

Injection and infusion of bacteria: two syringes for bolus injection and infusion were prepared with bacteria suspension with an approximate concentration of 5x108 CFU/mL. The bolus was given as 10 injections of 1 mL in 10 minutes. The infusion was immediately thereafter given as a continuous infusion of 0.3 mL/kg/h for 1 hour. Bolus injections and infusions were administered to the cephalic vein in the front leg and/or to the lateral auricular vein in the ear. T=0 of the experiment was defined as the start of the infusion. At each sampling occasion, three samples of blood were withdrawn (0.5 mL in a 1 mL syringe). The samples were placed on wet ice and processed as swiftly as possible. Three baseline blood samples were withdrawn from the animal before connecting the SangAsept system to the animal. Three blood samples, 0.5 mL in 1 mL syringe, were withdrawn from the inlet to (i.e., before or upstream) and outlet (i.e., after or downstream) from SangAsept at each subsequent occasion. The samples were directly placed on wet ice and processed as swiftly as possible. The blood was transferred to sterile, cold 1.5 mL microcentrifuge tubes. 100 µl of each blood sample was transferred to new microcentrifuge tubes containing 900 µl of ice-cold sterile PBS and mixed (1:10 dilution). Thereafter 100 µl of 1:10 dilution was transferred to new microcentrifuge tubes containing 900 µl of ice-cold sterile PBS and mixed (1:100 dilution). 100 µl of each sample: undiluted, 1:10 and 1:100 sample was directly transferred to a cysteine lactose electrolyte deficient (CLED) plate and spread. The plates were transferred to an incubator, 37°C, for overnight culture. Scoring of CFU: the number of CFU on each plate was counted and noted. Bacterial quantification was performed by quantitative bacterial determination of blood on CLED agar plates (Becton Dickinsson). 0.1 mL blood was aliquoted on agar plates and cultured at 37°C overnight and bacteria were quantified with viable count technique. Dialysis catheter blood samples 0.1 mL were collected upstream and downstream from the extracorporeal ozone system before initiation of the treatment and after the 15 minutes of treatment had ended and aliquoted on CLED plates in triplicates for bacteria quantification (CFU per mL).

### Immunology

The Luminex assay, Porcine Premixed Multi-Analyte Kit (RnD Systems), was run according to the manufacturer's instructions on a Bio-Rad Bio-Plex MAGPIX instrument. Briefly, the pig serum was diluted 1:2 with the calibrator diluent supplied with the kit. 50 µL of the diluted sample or standard mix was added per well of the 96 well sample plate. 50 µL of microparticle cocktail were then added and the plate incubated on a horizontal orbital shaker for 2 hours. The plate was then washed using a Bio-Rad Bio-Plex pro magnetic wash station, using the supplied wash buffer and the standard wash program. 50 µL of the Biotin-antibody cocktail were then added to the sample and standard wells and incubated on the orbital shaker for 1 hour. The plate was then washed again, same as before on the wash station, after which 50 µL of Streptavidin-PE solution was added and the plate incubated on the orbital shaker for 30 minutes. The plate was washed at the washing station and then the beads were resuspended and read on the Bio-Rad Bio-Plex MAGPIX instrument.

### Physiological calculations

Venous admixture (Qs/Qt) was calculated by the shunt equation (Berggren equation):

Qs/Qt = (CcO₂ - CaO₂) / (CcO₂ - CvO₂),

where Qs/Qt = shunt fraction (shunt flow divided by total cardiac output), CcO₂ = pulmonary end capillary O₂ content, same as alveolar O₂ content = SaO₂ x Hb x 1.3. SaO₂ was assumed to be 100% in the lungs. CaO₂ = arterial O₂ content (SaO₂ x Hb x 1.3), CvO₂ = mixed venous O₂ content (SvO₂ x Hb x 1.3). V'A/Q' = alveolar minute ventilation / cardiac output. Units: CcO₂/CaO₂ /CvO_{2 =} mL/L, Hb = g/L, SaO₂ = %.

### Statistical analyses

Statistical analyses were performed using GraphPad Prism version 10.3.0 for Windows (GraphPad Software, La Jolla, CA). The primary outcome was the concentration of viable *P*. *aeruginosaP. aeruginosa* in blood. P<.05 was considered statistically significant. Error bars represent the standard deviation. This study was a primary feasibility study which is why power calculation was not used. Temporal data sets were analyzed using a mixed-effects model (REML), fixed effects (type III) with Šídák's multiple comparisons test. We performed Shapiro-Wilk tests, confirming normality for all variables except MPAP, breathing frequency, and pO₂. However, residual analysis indicated these approximated normality. For complement pathways, two-tailed, paired t-tests were used. For cumulative resuscitation fluids, an unpaired t-test was performed. Comparison of survival curves was done by Log-rank (Mantel-Cox) test. *P*. *aeruginosa* before and after SangAsept was not normally distributed and the non-parametric Wilcoxon matched-pairs signed rank test was used.

### Results

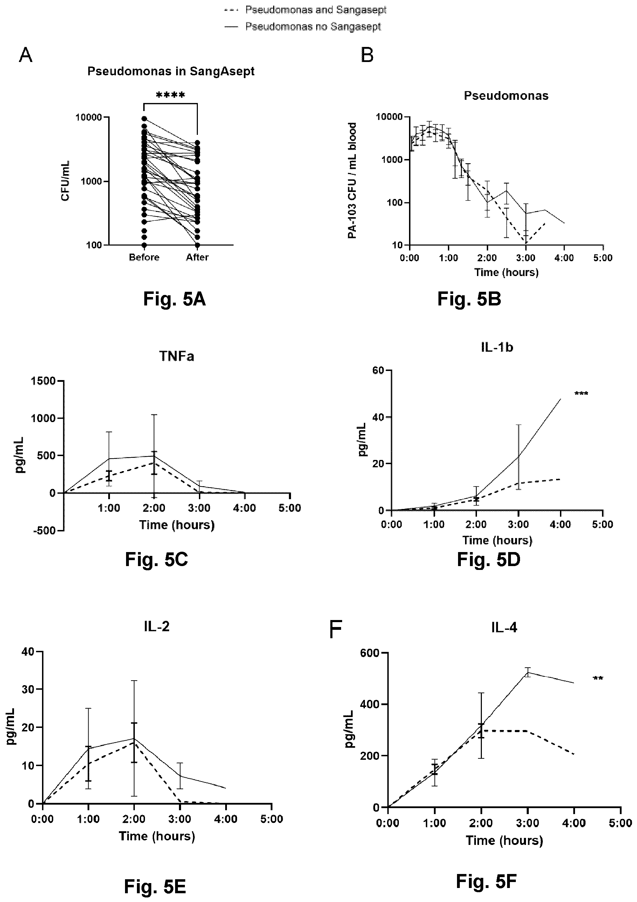

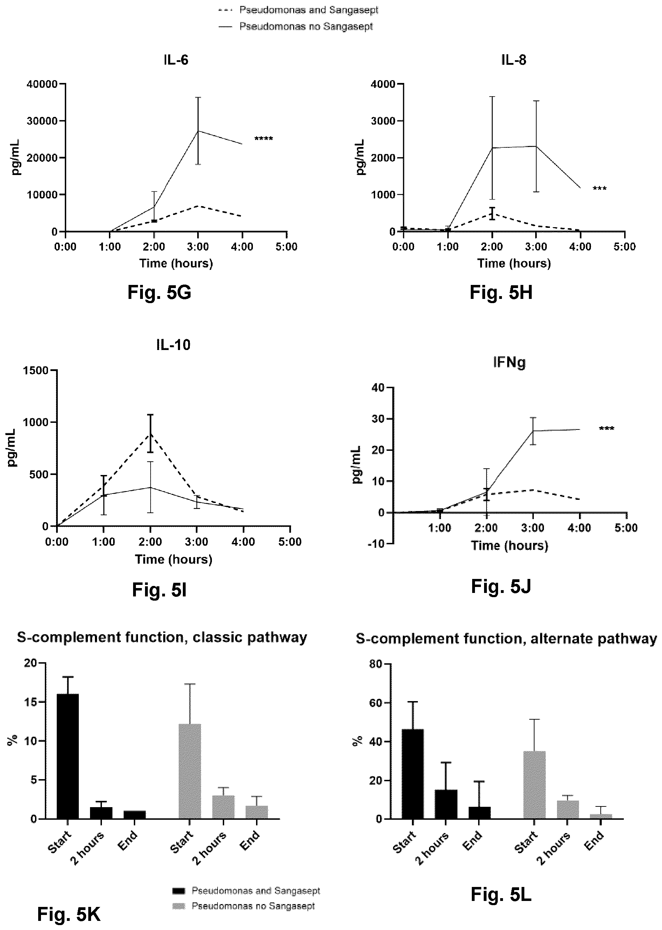

The mean (SD) duration of ozone treatment was 134 (67) minutes. A single pass treatment decreased viable *P*. *aeruginosa* by 53 %, mean 2193 to 1023 CFU/mL, mean of differences -1170 (95% CI -1689 to -651, P <0.0001) (Figure 5A). The bacterial bolus injection and infusion caused an increase in the concentration of viable bacteria during the first hour, after which a spontaneous decline was noted. No difference in bacterial concentration between groups was detected in peripheral venous blood (P=0.6751) (Figure 5B). TNFa did not differ (Figure 5C). IL-1β decreased (p<0.005) (Figure 5D). IL-2 did not differ (Figure 5E). IL-4 decreased (p<0.01) (Figure 5F). IL-6 decreased (p<0.001) (Figure 5G). IL-8 decreased (p<0.005) (Figure 5H). IL-10 did not differ (Figure 5I). IFN-γ decreased (p<0.005) (Figure 5J). Complement function, classic pathway and alternative pathway, decreased at 2 hours and end of experiments, compared to baseline. No differences were seen by ozone treatment (Figure 5K-L).

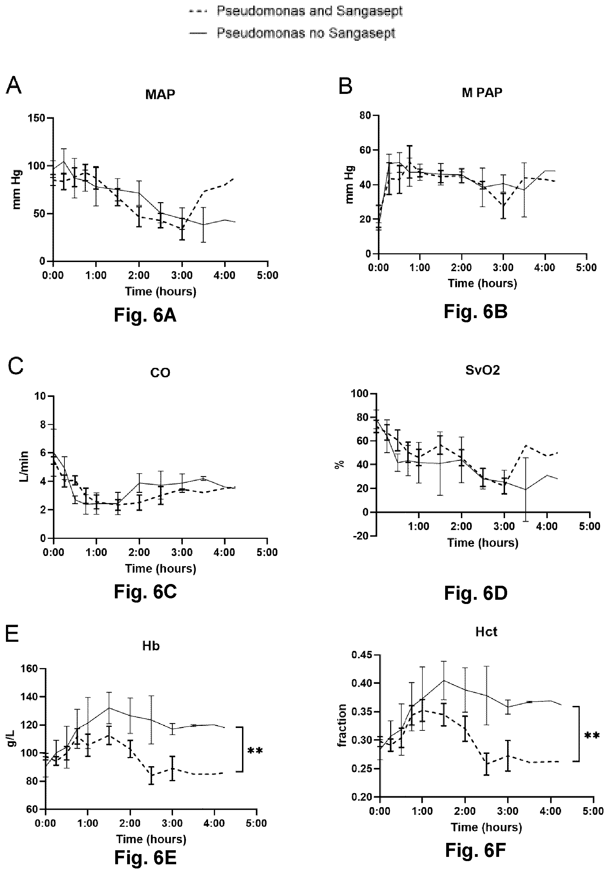

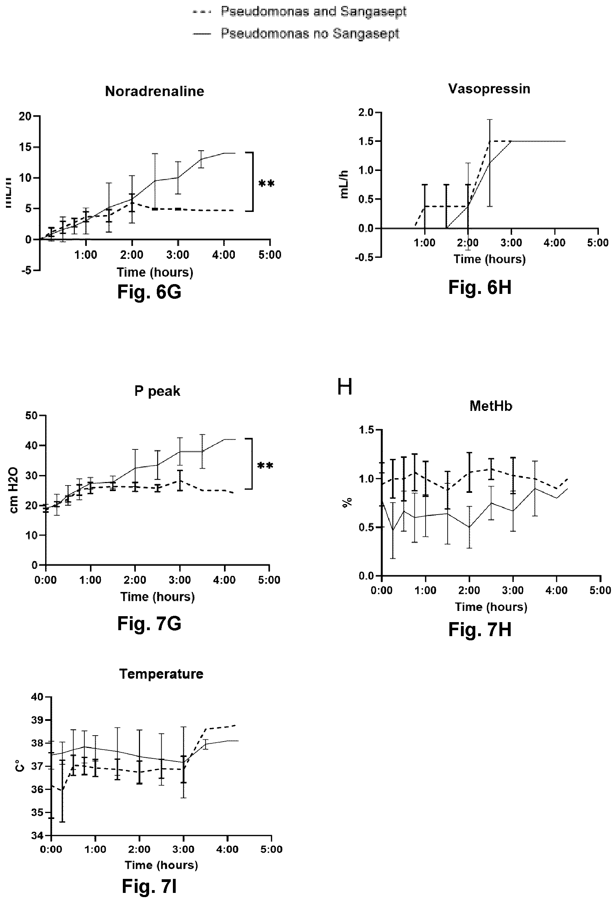

Circulatory effects from the sepsis induction and the ozone treatment were compared. MAP, MPAP, CO, SvO2 did not differ between groups (Figure 6A-D). Hb decreased in the ozone treatment group (p<0.01) (Figure 6E). Hct decreased (p<0.01) (Figure 6F). Noradrenalin doses to maintain blood pressure were lower in the ozone treatment group (p<0.01) (Figure 6G). Vasopressin doses did not differ between groups (Figure 6H). Median survival in ozone treatment was 134 minutes and no treatment 159 minutes. No difference was detected between groups (p=0.5843). Mean (SD) cumulative resuscitation with Ringers's acetate was 5767 (751) mL in the ozone treatment group and 8600 (2043) mL in the non-treatment group. No difference was detected between groups (p=0.0654).

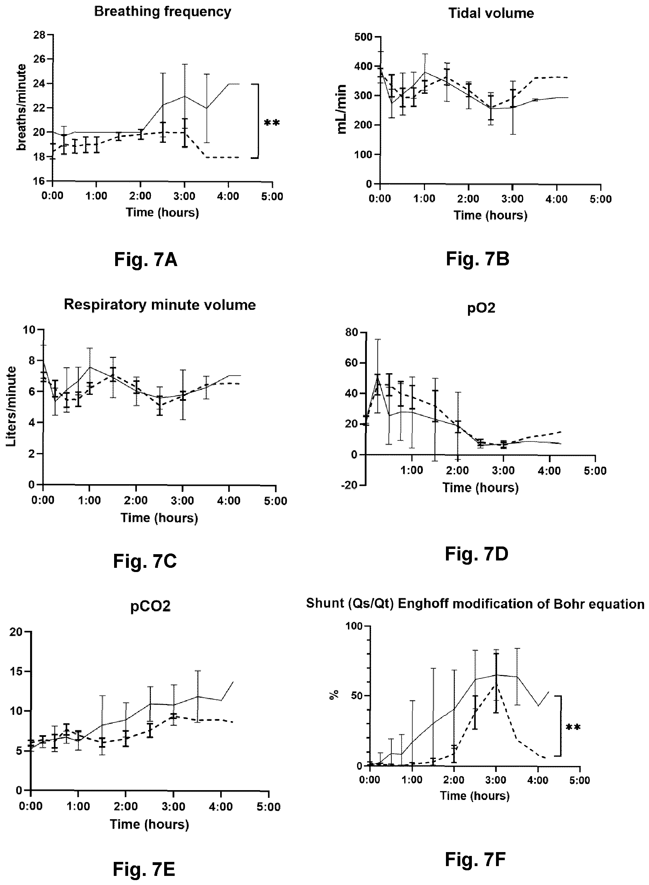

Respiratory effects from the sepsis induction and the ozone treatment were compared. Breathing frequency decreased in the ozone treatment group (p<0.01) (Figure 7A). Tidal volume (Figure 7B), respiratory minute volume (Figure 7C), PaO2 (Figure 7D) and PaCO2 (Figure 7E) did not differ between groups. Pulmonary shunt decreased in the ozone treatment group (p<0.01) (Figure 7F). PIP decreased in the ozone treatment group (p<0.01) (Figure 7G). MetHb did not differ between groups (Figure 7H). Core body temperature did not differ between groups (Figure 7I).

### Discussion

This study shows that that extracorporeal ozonation modulated the immune response in *P*. *aeruginosaP. aeruginosa* sepsis. The modulation was cytokine-specific and did not affect complement activation. In addition, indications of decreased vascular permeability and improved lung function were detected.

It was shown earlier that extracorporeal ozonation of blood decreased the amount of viable *E*. *coli,* but sensitivity to ozone may differ between strains. *P*. *aeruginosa* is a major cause of sepsis-derived mortality in hospitals and this study was conducted to investigate whether ozone would affect this strain. To simulate a clinical scenario where immediate and effective antibacterial treatment is critical, we employed a septic shock model using Pseudomonas aeruginosa infusion. Median survival in this model was under 160 minutes, despite optimal resuscitation, reflecting the severity of the condition and the high mortality associated with such infections. The bacterial infusion and study duration were informed by our prior experience and were designed to achieve measurable bacteremia. Ozone treatment was administered via a standard dialysis catheter inserted into the femoral vein. Treatment began 30 minutes prior to the bacterial infusion and continued for as long as conditions allowed. The system was compatible with an intensive care environment and did not induce any observable physiological adverse effects.

First, the effect on bacterial count was investigated. *P. aeruginosa* decreased with a rate of 53% by a single pass through the system, when measured in the blood flow before and after the chamber, which indicated that extracorporeal ozonation was able to decrease the number of viable bacteria. However, no difference was seen between groups when measuring bacteria in blood taken from a peripheral vein, i.e. blood that had been circulating in the swine, together with fluids, medical infusions and the bacterial infusion. To produce sufficient levels of bacteria in blood for accurate subsequent quantification in agar plates, a high infusion rate was chosen. This produced supra-normal levels of bacteria in the blood. It is possible that these levels surpassed the capacity of the ozonation system, and that lower levels would be manageable by the system. Moreover, while the magnitude of bacteremia may correlate with the severity of disease, other factors play more important roles in determining the patient's outcome. Most episodes of clinically significant bacteremia are characterized by low numbers of bacteria per millilitre of blood.

Next, the levels of circulating cytokines were quantified. Cytokines are a diverse group of small proteins, typically under 40 kDa, that include interleukins, chemokines, interferons, tumor necrosis factors, and growth factors. These molecules are primarily synthesized and secreted by immune cells. During an infection, the cytokine network is activated, consisting of both pro-inflammatory and anti-inflammatory cytokines. The interplay between these opposing regulatory pathways ultimately dictates the overall inflammatory response within the cytokine network. It was detected that IL-1β, IL-4, IL6, IL-8 and IFN-γ decreased by ozonation. IL-1β , IL6, IL-8 and IFN-γ are mostly proinflammatory while IL-4 is anti-inflammatory. The effect was consistent during the observation period and was specific to cytokine levels; the levels of complement factors were also quantified and no effect by ozonation was found. Ozone may stimulate the function of the peripheral blood cells.

Then, circulatory effects were investigated. MAP decreased and MPAP increased because of sepsis, which also caused CO and SvO2 to decrease. Interestingly, Hb and Hct decreased by ozonation, although the total amount of resuscitation with crystalloids did not differ between groups. It is possible that the decreased Hb and Hct were consequences of decreased vascular permeability - which retains water in the bloodstream and therefore decreases the relative concentration of Hb and subsequently Hct. A highly selective endothelial barrier is essential to maintain tissue fluid homeostasis and to support normal organ function. In response to cytokines produced by immune cells, the endothelium expresses adhesion molecules and produces vasoactive compounds, inflammatory cytokines, and chemoattractants, which can lead to increased capillary permeability. A main feature of the endothelium in sepsis is increased permeability or loss of barrier function, resulting in a shift of circulating elements and tissue oedema. Thus, it is possible that the decreased cytokine concentration in the blood caused a clinically measurable decreased vascular permeability, which should be confirmed in future investigations.

Then, respiratory effects were investigated. The ozonation group had decreased breathing frequency and peak airway pressures required to maintain respiratory minute volumes, and equal PaO₂ and PaCO₂. The lung is the earliest and most susceptible target organ in multiple organ dysfunction caused by sepsis, and up to 50% of sepsis patients experience acute lung injury (ALI). Patients with sepsis-induced ALI have weakened gas exchange function due to lung inflammation and tissue damage and pathological processes include pulmonary vascular endothelial damage, reduced alveolar surface tension, inflammatory factor release and pulmonary interstitial fibrosis. Pulmonary shunt was then calculated. It decreased in the ozonation group, thus improving lung function. Pulmonary shunt refers to the passage of venous blood into the arterial blood system by passing the alveoli-blood gas exchange. Pulmonary shunting is a well-defined drop in the physiologic coupling of lung ventilation and lung perfusion in ALI. Whether this also is a consequence of endothelial improved function remains to be elucidated.

Methemoglobin levels did not increase, consistent with findings from our previous investigation. Methemoglobin is an oxidized form of hemoglobin (Hb), typically maintained at less than 1% of total hemoglobin under normal conditions. Clinically significant methemoglobinemia can arise from exposure to oxidizing agents, including certain medications. The oxidative stress induced by ozone and its bactericidal effects must be balanced against potential adverse effects on blood by ensuring ozone doses remain within the neutralizing capacity of the blood's antioxidative defenses. In this study, the ozone dose may not have caused excessive oxidative damage.

The ozonation system comprises a new method of modifying the immune response in sepsis. Immunomodulatory therapy in managing sepsis, a condition marked by a dysregulated immune response to infection, is likely pivotal for improving mortality. The endothelium undergoes dramatic changes during sepsis and remains one of the most compelling targets for therapeutic development. However, the modalities recommended for the management of sepsis do little to protect the endothelium or restore endothelial function, leaving much room for future drug development. Integrating such therapies into sepsis management protocols, particularly for patients with severe or refractory disease, holds the potential to reduce mortality.

### Conclusion

Extracorporeal ozone blood treatment modulated the immune response in P. *aeruginosa* septic shock, which decreased cytokines and was associated with indications of decreased vascular permeability and improved lung function.

## Claims

1. A gas composition comprising oxygen and/or ozone for use in treatment to reduce inflammation in conditions **characterized by** dysregulated immune response.

2. The gas composition for use according to claim 1, wherein said gas composition comprises 0-20% ozone, preferably 0 - 10% ozone, and from 100% to 80% oxygen.

3. The gas composition for use according to claim 1, wherein such gas composition consists of oxygen and/or ozone.

4. The gas composition for use according to any of the previous claims, wherein said use reduces one or more pro-inflammatory cytokines, thereby reducing inflammation.

5. The gas composition for use according to claim 4, where said cytokines are selected from the group consisting of IL-1β, IL-6, IL-8, TNF-α and IFN-γ.

6. The gas composition for use according to any of claims 4 or 5, wherein said one or more cytokines are reduced by at least 1%, such as at least 5%, such as at least 8%, at least 10%, at least 12%, or for example in the range from 1 to 80%, such as 5 to 80%.

7. The gas composition for use according to any one of the preceding claims, wherein said conditions **characterized by** dysregulated immune response may be either secondary to an infectious trigger, or secondary to a non-infectious trigger.

8. The gas composition for use according to claim 7, wherein the non-infectious trigger is selected from the group consisting of trauma, tissue damage, multiple sclerosis, autoimmune and low-grade inflammatory conditions.

9. A gas composition comprising oxygen and/or ozone for use in treatment to preserve organ function in conditions **characterized by** dysregulated immune response.

10. A gas composition comprising oxygen and/or ozone for use in treatment to protect the endothelium or restore endothelial function in conditions **characterized by** dysregulated immune response.

11. The gas composition for use according to any of the preceding claims, wherein said use comprises at least one additional agent such as one or more selected from the group consisting of one or more antimicrobial agents and/or immunomodulators.

12. The gas composition for use according to any of the preceding claims, wherein said gas composition is administered/contacted with blood by means of an apparatus comprising an ozonation chamber (2), a blood flow inlet (2a), and a blood flow outlet (2b).

13. The gas composition for use according to claim 12, wherein the apparatus comprises:
an ozonation chamber (2), a blood flow inlet (2a), and a blood flow outlet (2b) wherein,
said ozonation chamber (2) comprises an ozone injector (3) , said ozone injector (3) configured for connection to a source of ozone containing gas composition and to inject bubbles of ozone containing gas into a flow of blood through the ozonation chamber (2), wherein the flow of blood is vertically upwards through the blood ozonation chamber (2) during injection,
said ozonation chamber (2) and said ozone injector (3) are configured such that the vertically upwards flow of blood through the blood ozonation chamber (2) entrains said bubbles of ozone containing gas into said flow of blood,
wherein,
a blood processing unit (13) comprising means for removing foam and bubbles from the flow of blood downstream the ozone injector (3),
said ozone injector (3) is a porous ozone injector, and
said ozone injector is configured to inject microbubbles having a mean diameter of less than 5 µm.

14. The gas composition for use according to claim 13, wherein extracorporeal treatment of blood may be performed continuously, or in intermittent periods.

15. A gas composition for use comprising oxygen and/or ozone for use in treatment to reduce inflammation in conditions **characterized by** dysregulated immune response, wherein said gas comprises 0-10% ozone, wherein said one or more pro-inflammatory cytokines selected from the group consisting of IL-1β, IL-6, IL-8, TNF-α and IFN-γ, by at least 1%,
optionally comprising one or more further agents selected from the group consisting of one or more antimicrobial agents and/or immunomodulators wherein said gas is administered/contacted with blood by means of an apparatus,
an ozonation chamber (2), a blood flow inlet (2a), and a blood flow outlet (2b)
wherein,
said ozonation chamber (2) comprises an ozone injector (3) , said ozone injector (3) configured for connection to a source of ozone containing gas and to inject bubbles of ozone containing gas into a flow of blood through the ozonation chamber (2), wherein the flow of blood is vertically upwards through the blood ozonation chamber (2) during injection,
said ozonation chamber (2) and said ozone injector (3) are configured such that the vertically upwards flow of blood through the blood ozonation chamber (2) entrains said bubbles of ozone containing gas into said flow of blood,
wherein,
a blood processing unit (13) comprising means for removing foam and bubbles from the flow of blood downstream the ozone injector (3),
said ozone injector (3) is a porous ozone injector, and
said ozone injector is configured to inject microbubbles having a mean diameter of less than 5 µm,
thereby reducing inflammation.
